(19) Europäisches Patentamt
European Patent Office
Office européen des brevets

(11) EP 4 431 602 A1

(12) EUROPEAN PATENT APPLICATION
published in accordance with Art. 153(4) EPC

(43) Date of publication:
**18.09.2024 Bulletin 2024/38**

(21) Application number: **22892975.8**

(22) Date of filing: **26.07.2022**

(51) International Patent Classification (IPC):
***C12N 9/64*** *(2006.01)* ***C07K 14/33*** *(2006.01)*
***A61K 9/16*** *(2006.01)* ***A61K 38/48*** *(2006.01)*
***A61K 8/02*** *(2006.01)* ***A61K 8/66*** *(2006.01)*

(52) Cooperative Patent Classification (CPC):
**A61K 8/02; A61K 8/66; A61K 9/16; A61K 38/48; C07K 14/33; C12N 9/64**

(86) International application number:
**PCT/KR2022/010991**

(87) International publication number:
**WO 2023/085552 (19.05.2023 Gazette 2023/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **11.11.2021 KR 20210155208**

(71) Applicant: **Inibio Co., Ltd.**
**Bucheon-si, Gyeonggi-do 14445 (KR)**

(72) Inventors:
- **KIM, Chung Sei**
  **Seoul 07651 (KR)**
- **YIM, Hyeon A**
  **Siheung-si, Gyeonggi-do 15010 (KR)**
- **KIM, Young Je**
  **Bucheon-si, Gyeonggi-do 14539 (KR)**
- **AN, Yeong Duk**
  **Bucheon-si, Gyeonggi-do 14511 (KR)**

(74) Representative: **Meissner Bolte Partnerschaft mbB**
**Patentanwälte Rechtsanwälte**
**Postfach 86 06 24**
**81633 München (DE)**

# (54) DRYING PROCESS OF BOTULINUM TOXIN FORMULATION

(57) The present invention relates to a method for drying botulinum toxin under reduced pressure. In the case of using a method for preparing a botulinum toxin dried cake according to this method for drying under reduced pressure, the drying time can be significantly shortened while maintaining the efficacy of the botulinum toxin.

Fig. 1

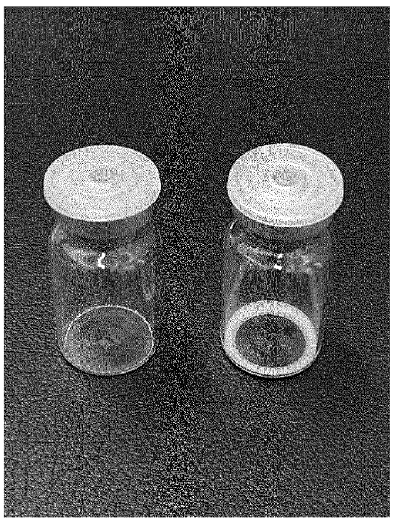

EP 4 431 602 A1

## Description

### TECHNICAL FIELD

The present invention relates to a drying process for a toxin formulation, and specifically, to a method for preparing a botulinum toxin dried cake by a reduced pressure drying method.

### BACKGROUND ART

Botulinum toxin (BTX) is a neurotoxin produced by an anaerobic bacterium called *Clostridium botulinum (C. botulinum).* There exists a total of 7 types of botulinum toxin (types A to G), of which two types, i.e., botulinum type A (BTX-A) and type B (BTX-B), have been purified and are being medically used.

Botulinum toxin blocks muscle contraction signal transduction to relax muscles by inhibiting the release of acetylcholine, a neurotransmitter. In other words, it blocks the release of acetylcholine, a neurotransmitter secreted from the presynaptic terminal of the neuromuscular junction, causing nerve paralysis. While fillers are medical materials that fill an area with insufficient skin volume, botulinum toxin preparation differs from fillers in that it is a medication containing ingredients that deteriorate activity of muscle by blocking the release of neurotransmitters that cause muscle contraction.

Botulinum toxin is mainly used to suppress or remove wrinkles between the eyebrows and around the eyes, as well as to treat upper limb stiffness of stroke, eyelid spasms, equinus deformity, etc. Botulinum toxin is gradually expanding its scope of indications.

Commercially available botulinum toxin products may be provided in solution form with excipients containing sodium chloride and human serum albumin or in solid form (i.e., dried cakes) after a drying process. Most botulinum toxin manufacturers produce products that have undergone a freeze-drying process. For example, there are Meditoxin®, Xeomin®, Dysport®, etc.

Meanwhile, in the drying process of botulinum toxin, many conventional freeze drying methods have been known (such as KR 10-2012-0112248 A, etc.), but the methods take quite a long time (approximately 18 to 48 hours) since they essentially involve the freezing process and moisture is removed through the sublimation process. In particular, the freezing process, which is an essential process of the freeze-drying process, may cause a decrease in activity through damage to the protein structure due to ice nuclei formation or an imbalance of excipient concentration that occurs partially during freezing.

This freeze-drying process is generally known as an advanced type of drying method for dry formulations of protein pharmaceuticals. However, it is unsuitable for botulinum toxin formulations in which extremely small amounts of protein are used.

Therefore, there is a need to develop a drying process suitable for the characteristics of botulinum toxin formulations. In addition, a method is needed to efficiently use the long process time for primary and secondary drying due to the characteristics of the freeze-drying process.

### DETAILED DESCRIPTION

### TECHNICAL OBJECTIVE

The inventors conducted an intensive research to develop an optimized, reduced pressure drying method for botulinum toxin that maintains effectiveness and stability. As a result, the inventors successfully demonstrated that by efficiently controlling parameters related to the drying process, such as pressure and (shelf) temperature, the protein botulinum toxin can be protected from external stimuli occurring during the process and the drying time can be significantly shortened.

Therefore, the purpose of the present invention is to provide a method for preparing a botulinum toxin dried cake.

### MEANS FOR SOLVING THE PROBLEMS

The inventors conducted an intensive research to develop an optimized, reduced pressure drying method for botulinum toxin that maintains effectiveness and stability. As a result, the inventors successfully demonstrated that by efficiently controlling parameters related to the drying process, such as pressure and (shelf) temperature, the protein botulinum toxin can be protected from external stimuli occurring during the process and the drying time can be significantly shortened.

The present invention relates to a method for preparing a botulinum toxin dried cake.

Hereinafter, the present invention will be discussed in more detail.

According to one aspect of the present invention, the present invention provides a method for preparing a botulinum toxin dried cake, comprising the step of drying botulinum toxin under reduced pressure at a pressure of 1,500 to 60,000 mTorr and a temperature of 3°C to 25°C.

As used herein, the term "botulinum toxin (BTX)" refers to a toxin that can be obtained by purifying it from *a Clostridium botulinum (C. botulinum)* strain. The botulinum toxin produced by *Clostridium botulinum (C.botulinum)* strains has a size of about 150 kDa. A botulinum toxin complex, which is a complex of botulinum toxin and one or more non-toxin proteins, has a size of about 300 kDa to 900 kDa.

The above botulinum toxin may be used for various medical purposes, as well as suppressing or improving wrinkles between the eyebrows and those around the eyes.

As used herein, the term "cake" refers to a dried material on the bottom surface of a vial in dry formulation, which may be used separately from a powder formulation.

In one embodiment of the present invention, a strain of the present invention producing botulinum toxin is *Clostridium botulinum (C.botulinum).* More specifically, the clostridium botulinum Type A (ATCC 19397) strain may be used, but the strain of the present invention is not limited thereto. Botulinum toxin type A has been approved by the U.S. Food and Drug Administration (FDA) for the treatment of essential blepharospasm, strabismus, and hemifacial spasm in patients over 12 years of age. It also has been approved for the treatment of cervical dystonia, glabellar (facial) wrinkles, and hyperhidrosis.

In another embodiment of the present invention, various conventionally known processes may be used to produce the botulinum toxin of the present invention, and the process for the present invention is not particularly limited.

Specifically, for example, it may involve a strain culture medium producing botulinum toxin that has been cultured, and subjected to precipitation, filtering, re-dissolution, and purification processes once or at least two times.

More specifically, for example, the botulinum toxin of the present invention may be a strain culture medium producing botulinum toxin prepared by purifying it into a crystalline complex which consists of an active high molecular weight toxin protein and a relevant erythrocyte agglomerate protein, through a series of acid precipitations of a botulinum toxin complex from a culture of *Clostridium botulinum (C. botulinum)* cultured in a specific medium obviously understood by those skilled in the art as suitable for culturing *Clostridium botulinum* (*C. botulinum),* and dissolving the purified crystalline complex in a solution comprising brine and a stabilizer.

The culture conditions of the strain culture medium producing botulinum toxin may be appropriately adjusted based on the general knowledge of a person skilled in the relevant art depending on the culture environment.

In another embodiment of the present invention, the concentration of the botulinum toxin of the present invention may be 250 U/mL to 5,000 U/mL.

Specifically, in a preferred embodiment of the present invention, the concentration of the botulinum toxin may be 250 U/mL to 5,000 U/mL, 300 U/mL to 5,000 U/mL, 400 U/mL to 5,000 U/mL, 500 U/mL to 5,000 U/mL, 600 U/mL to 5,000 U/mL, 700 U/mL to 5,000 U/mL, 800 U/mL to 5,000 U/mL, 900 U/mL to 5,000 U/mL, 1,000 U/mL to 5,000 U/mL, 1,000 U/mL to 4,500 U/mL, 1,000 U/mL to 4,000 U/mL, 1,000 U/mL to 3,500 U/mL, 1,000 U/mL to 3,000 U/mL, 1,000 U/mL to 2,500 U/mL, 1,000 U/mL to 2,000 U/mL, 1,000 U/mL to 1,500 U/mL, 1,000 U/mL to 1,200 U/mL, 800 U/mL to 1,200 U/mL, 800 U/mL to 1,100 U/mL, 800 U/mL to 1,000 U/mL, 800 U/mLto 900 U/mL, 900 U/mLto 1,200 U/mL, 1,000 U/mLto 1,200 U/mL, or 1,100 U/mL to 1,200 U/mL.

In another embodiment of the present invention, the method for preparing the botulinum toxin dried cake of the present invention may involve drying the above-described botulinum toxin under reduced pressure at a pressure of 1,500 or more and less than 60,000 mTorr.

Specifically, in a preferred embodiment of the present invention, the drying under reduced pressure of the present invention may be performed at a pressure of 1,500 to 60,000 mTorr, 1,500 to 55,000 mTorr, 1,500 to 50,000 mTorr, 1,500 to 45,000 mTorr, 1,500 to 40,000 mTorr, 1,500 to 35,000 mTorr, 1,500 to 30,000 mTorr, 1,500 to 25,000 mTorr, 1,500 to 20,000 mTorr, 1,500 to 15,000 mTorr, 1,500 to 14,000 mTorr, 1,500 to 13,000 mTorr, 1,500 to 12,000 mTorr, 1,500 to 11,000 mTorr, 1,500 to 10,000 mTorr, 1,500 to 9,000 mTorr, 1,500 to 8,000 mTorr, 1,500 to 7,000 mTorr, 1,500 to 6,000 mTorr, 1,500 to 5,000 mTorr, 1,500 to 4,000 mTorr, 1,500 to 3,500 mTorr, 1,500 to 3,000 mTorr, 1,500 to 2,500 mTorr, 1,500 to 2,000 mTorr, 2,500 to 3,000 mTorr, 2,500 to 3,500 mTorr, 2,500 to 4,000 mTorr, 2,500 to 4,500 mTorr, 2,500 to 5,000 mTorr, 2,500 to 6,000 mTorr, 2,500 to 7,000 mTorr, 2,500 to 8,000 mTorr, 2,500 to 9,000 mTorr, 2,500 to 10,000 mTorr, 2,500 to 11,000 mTorr, 2,500 to 12,000 mTorr, 2,500 to 13,000 mTorr, 2,500 to 14,000 mTorr, 2,500 to 15,000 mTorr, 2,500 to 20,000 mTorr, 2,500 to 25,000 mTorr, 2,500 to 30,000 mTorr, 2,500 to 35,000 mTorr, 2,500 to 40,000 mTorr, 2,500 to 45,000 mTorr, 2,500 to 50,000 mTorr, 2,500 to 55,000 mTorr, or 2,500 to 60,000 mTorr.

If it deviates from the above ranges, protein damage may occur due to phenomena such as boiling over, and a perfectly dried product may not be obtained because sufficient drying is not feasible.

Meanwhile, in the above drying under reduced pressure, it is necessary to reach the target pressure at a constant speed in a stably controlled state from normal [atmospheric] pressure, which is general atmospheric conditions.

In another embodiment of the present invention, the method for preparing the botulinum toxin dried cake of the present invention may involve drying the above-described botulinum toxin under reduced pressure at a temperature of 3 to 25°C.

Specifically, in a preferred embodiment of the present invention, the drying under reduced pressure of the present invention may be performed at a temperature of 3°C to 25°C, 5°C to 25°C, 7°C to 25°C, 9°C to 25°C, 11°C to 25°C, 12°C to 25°C, 3°C to 20°C, 5°C to 20°C, 7°C to 20°C, 9°C to 20°C, 11°C to 20°C, 12°C to 20°C, 3°C to 18°C, 3°C to 16°C, 3°C to 14°C, or 3°C to 12°C.

If it deviates from the above ranges, due to higher or lower temperatures, the structural instability of the botulinum toxin may increase or physical damage and deformation thereto may occur, which may lead to a decrease in efficacy.

In another embodiment of the present invention, the method for preparing the botulinum toxin dried cake of the present invention may be performed until the humidity of the botulinum toxin dried cake of the present invention reaches 3% or less.

Specifically, the method for preparing the botulinum toxin dried cake of the present invention may involve drying the above-described botulinum toxin under reduced pressure for 0.5 hours or more, preferably 0.5 to 4 hours.

Specifically, in a preferred embodiment of the present invention, the drying under reduced pressure of the present invention may be performed in the range of 0.5 hours to 4 hours, 0.5 hours to 3 hours, 0.5 hours to 2 hours, 0.5 hours to 1 hour, 1 hour to 4 hours, 2 hours to 4 hours, or 3 hours to 4 hours.

The botulinum toxin dried cake, i.e., the final product prepared by the method for preparing a botulinum toxin dried cake of the present invention, may have a standard titer of 80% to 120, or 85% to 115%.

In the process of manufacturing the botulinum toxin dried cake of the present invention, depending on volume or surface area of a solvent for drying the product, boiling over may occur due to the amount of dissolved oxygen or change in concentration of the composition concentrated during the drying process, and unexpected ice crystal formation may occur due to changes in freezing point. If the product is dried under these conditions, the product quality may be deteriorated due to the irregularity in the product's properties and protein damages. Therefore, it is preferable for the drying process to be performed under the conditions that volume or surface area of a solvent for drying the product is appropriately adjusted, which can be easily controlled and performed by a person skilled in the art who understood the present specification.

The method for preparing a botulinum toxin dried cake of the present invention utilizes the above-described drying process under reduced pressure, by which the conventional freeze-drying process can be disused. In one aspect of the present invention, the present invention uses a drying process under reduced pressure, which shortens the processing time and minimizes damages to the protein drug product during drying. Accordingly, the present invention has the advantages in that it is more efficient and commercially more feasible compared to the conventional technologies.

## WORKING EFFECT OF THE INVENTION

The present invention relates to a method for drying botulinum toxin under reduced pressure. When the method for preparing a botulinum toxin dried cake of the present invention by drying under reduced pressure is used, the drying time can be significantly shortened while the efficacy of the botulinum toxin is maintained.

## BRIEF DESCRIPTION OF DRAWINGS

Fig. 1 demonstrates the difference in cake properties between reduced pressure-dried product and freeze-dried product.

Figs. 2a and 2b demonstrate the possibility of denaturation and loss of botulinum toxin according to conventional low vacuum, reduced pressure drying conditions.

Figs. 3a and 3b demonstrate the difference in cake properties (Fig. 3a) and the change in temperature within the vial (Fig. 3b) according to pressure conditions in the range of 1,000 to 70,000 mTorr in the drying under reduced pressure of the present invention.

Fig. 4 demonstrates the change in temperature within the vial according to pressure conditions in the range of 50,000 to 70,000 mTorr in the drying under reduced pressure of the present invention.

## MODES FOR CARRYING OUT THE INVENTION

Hereinafter, the present invention will be described in more detail based on the working examples. The working examples are presented merely in order to elaborate the present inventive concept more specifically, and it should be obvious to a person skilled in the art that in view of the gist of the present invention, the scope of the present invention should not be construed as being limited to the working examples as presented.

Unless otherwise indicated in the examples, "toxin" or "botulinum toxin" refers to the botulinum toxin type A complex with a molecular weight of about 900 kDa. The method disclosed herein has the applicability prepared for formulations of toxins, complexes, botulinum toxin serotypes, and botulinum neurotoxin components of about 150 kDa, about 300 kDa, and about 500 kDa as well as other molecular weights.

**Preparation Example: Preparation of a final stock solution of botulinum toxin**

The final stock solution of botulinum toxin was prepared by adding 0.9% sodium chloride (Merck, 1.37017.5000), 0.5% human serum albumin (Green Cross, 161B19508), and 1000U/mL botulinum toxin type A stock solution (Inibio Co., Ltd., Korea).

**Comparative Example 1: Comparison of titer of botulinum toxin with freeze-drying**

**1-1. Drying experiment**

Formulations dried under freeze-drying conditions and those dried under reduced pressure drying conditions were compared, respectively.

Freeze-drying conditions and reduced pressure drying conditions are indicated in Table 1 below.

[Table 1]

| Drying method | Process Name | Shelf temperature(°C) | Time(m) | Pressure (mTorr) |
|---|---|---|---|---|
| Freeze-drying | Freezing | -50 | 180 | |
| | 1st Drying | -50 | 480 | 200 |
| | 2nd Drying | 20 | 180 | 100 |
| Reduced pressure drying | Drying | 11.5 | 60 | 2500 |

As shown in Fig. 1, in the case of freeze drying (right vial in Fig. 1), a thicker white dried cake was formed compared to reduced pressure drying (left vial in Fig. 1). This dried cake was easily breakable due to the impact applied to the vial, and the shattering of the broken dried cake could occur.

**1-2. Titer experiment**

Regarding the final stock solution of botulinum toxin prepared in the above preparation example, the formulation dried under freeze drying conditions (*see* Table 2 below) was dissolved in 2.8 mL of physiological saline and administered to the abdominal cavity (0.1 mL/mouse) of ten ICR-mice (4 weeks old, body weight 18-22 g; Coretech Co., Ltd., Korea) respectively. The numbers of dead animals and living animals were checked for 3 days. Titers were calculated using a statistical program (CombiStats 6.1, EDQM). The appropriate titer standard was set to a range that includes the error range of animal testing (*see* Table 3 below). The results are shown in Table 4 below.

[Table 2]

| Process Name | Shelf temperature(°C) | Time (m) | Pressure (mTorr) |
|---|---|---|---|
| Freezing | -50 | 180 | |
| 1st Drying | -50 | 480 | 200 |
| 2nd Drying | 20 | 180 | 100 |

[Table 3]

| Experiment | Standard Titer Range(%) |
|---|---|
| Titer experiment | 100±15% |

[Table 4]

| Vial No. | Drying condition | Standard Titer (%) |
|---|---|---|
| # 1 (Comparative Example 1) | Freeze drying | 65.0 |
| #2 (Comparative Example 2) | Freeze drying | 56.3 |
| #3 (Comparative Example 3) | Freeze drying | 56.5 |

As shown in Table 4, the titer of the botulinum toxin formulations to which a conventional freeze drying process was performed was 56-65%, which is outside the range shown in Table 3 above, indicating that the efficacy was reduced due to protein damages.

The above results indicate that the formulation of botulinum toxin dried under reduced pressure can retain superior activity of botulinum toxin compared to the freeze-dried formulation.

**Reference Example: Confirmation of denaturation and loss of botulinum toxin due to reduced pressure drying**

The final stock solution of botulinum toxin prepared in the above preparation example was filled into vials by 5 mL or 1 mL, and the physical changes and freezing point changes depending on changes in conventional low-vacuum drying pressure were examined.

Specifically, the reduced pressure drying was performed by changing the pressure from 1 bar to 0.0019 bar (about 1500 mTorr). The shelf temperature was set to 5°C. Distilled water (DW) and 0.9% NaCl solution were used as control groups.

Fig. 2a shows the phase changes by pressure after 5 mL of each solution was filled into a vial. Under the above conditions, the boiling phenomenon of the solution occurred in the final stock solution of botulinum toxin below 10,000 mTorr, but it did not occur in the control group.

Fig. 2b shows the phase changes by pressure after 1 mL of each solution was filled into a vial. Under the above conditions, the boiling over and ice crystal formation of the final stock solution occurred at a pressure of 1,500 mTorr, but they did not occur in the control group.

This phenomenon is understood to have been occurred because volume or surface area of the solvent was not suitable for the characteristics and drying conditions of the solvent. This may make it difficult to properly control the drying process or cause sublimation rather than complete evaporation due to ice crystals generated during the process, making it difficult to perform a normal reduced pressure drying. In addition, if drying of the product proceeds under uncontrollable conditions as above, the product quality may be deteriorated due to nonuniformity of product properties and damages to proteins.

**Example 1: Establishment of reduced pressure drying conditions based on the titer of botulinum toxin**

For the formulation in which 0.1 mL of the final stock solution of botulinum toxin prepared in the above preparation example was filled into vials and reduced pressure drying was conducted under the conditions shown in Table 5 below, the titer was calculated in the same manner as in Comparative Example 1-2. The appropriate titer standard was set to a range that includes the error range of animal testing (*see* Table 3 below), and the results are shown in Table 5 below.

[Table 5]

| Vial No. | Pressure (mTorr) | Shelf Temperature (°C) | Standard Titer (%) |
|---|---|---|---|
| # 4 | 1500 | 3 | 99.5 |
| # 5 | 1500 | 3 | 97.7 |
| # 6 | 2500 | 3 | 108.8 |
| # 7 | 3500 | 3 | 113.5 |
| # 8 | 3500 | 5 | 102.8 |
| # 9 | 1500 | 12 | 84.4 |
| # 10 | 2500 | 11.5 | 94.8 |
| # 11 | 3500 | 11.5 | 105.8 |

(continued)

| Vial No. | Pressure (mTorr) | Shelf Temperature (°C) | Standard Titer (%) |
|---|---|---|---|
| # 12 | 1500 | 20 | 91.6 |
| # 13 | 2500 | 20 | 95.1 |
| # 14 | 3500 | 20 | 100.4 |
| # 15 | 3500 | 20 | 105.9 |
| # 16 (Comparative Example 4) | 500 | 25 | 81.6 |
| # 17 (Comparative Example 5) | 70000 | 25 | 91.0 |

As shown in Table 5, the titer of the botulinum toxin formulation that was dried for 1 hour at 1,500 to 30,000 mTorr and 3 to 25 °C satisfied the range shown in Table 3. On the other hand, when the drying process was performed in a range outside 1,500 to 30,000 mTorr (Comparative Examples 4 and 5), it was found that the titer of the botulinum toxin formulation did not satisfy the appropriate range.

**Example 2: Drying time under the reduced pressure drying conditions of the present invention #1**

Generally, drying can be performed in two steps: the separation of water molecules connected by hydrogen bonds, and the removal of water molecules variously interacting with other excipients and proteins that make up the final stock solution. When water molecules form bonds with each other, they can be separated with relatively less energy than water molecules interacting with other molecules. In most cases, water molecules are bonded to each other, and primary evaporation is performed by separating them. In this process, the liquid product shows a process of changing into a gaseous phase. Secondary evaporation occurs when the bonds in the interaction between water molecules and other excipients are broken, and it is determined that drying is completed if this process is almost completed. As a result of humidity measurement, the humidity must be within 3% to meet the standards for dried cake injections.

Therefore, the speed of moisture evaporation or suitability can be confirmed through the phase change of the product.

**2-1. Time for phase change (liquid → gas) at each pressure condition**

Based on the results of Example 1, the empty vials were filled with the final stock solution of the botulinum toxin prepared in the above preparation example by 0.1 mL each, and time for the phase change at each pressure condition was examined.

Specifically, as shown in Table 6 below, the drying process was performed for 1 hour at conditions of 1,000 to 70,000 mTorr and 25°C. Change in temperature due to the phase change was measured by a temperature sensor installed in the vial. The speed at which the phase change occurs was faster with lower pressure, by which it was confirmed that evaporation was occurring.

[Table 6]

| Vial No. | Pressure (mTorr) | Time for phase change (min) |
|---|---|---|
| # 18 (Comparative Example 6) | 1,000 | 11 |
| # 19 | 3,000 | 13 |
| # 20 | 10,000 | 15 |
| # 21 | 30,000 | 43 |
| # 22 | 50,000 | - |
| # 23 | 60,000 | - |
| # 24 (Comparative Example 7) | 70,000 | - |

As shown in Table 6 and Figs. 3a and 3b, a white dried cake was formed on the bottom of most vials, which results from the phase change due to evaporation. The time taken for the phase change to occur was found to be 11 to 43 minutes. However, at 50,000 mTorr, 60,000 mTorr and 70,000 mTorr, this phenomenon was not observed within 1 hour.

The above results suggest that at higher pressures, the time required for complete drying (evaporation) will increase, and that preparation of the botulinum toxin dried cake pursued by the present invention may be difficult at pressures exceeding a certain range (Comparative Example 7).

**2-2. Humidity of the botulinum toxin dried cake as produced**

Based on the results of Example 1 and 2-1, the empty vials were filled with the final stock solution of the botulinum toxin prepared in the above preparation example by 0.1 mL each. For the vials containing the botulinum toxin dried cake that was dried under reduced pressure at 3,500 mTorr and 5°C, the moisture was measured in an environment with a humidity of 10% or less. The humidity (%) was calculated using the following calculation formula, and the appropriate humidity standards are shown in Table 7 below with reference to the Pharmacopoeia.

[Formula]

$$\text{Humidity (\%)} = \frac{\text{Moisture content of botulinum toxin dried cake} - \text{Moisture content of empty vial}}{\text{Weight of botulinum toxin dried cake}} \times 100\ \%$$

[Table 7]

| Experiment | Range | Reference |
|---|---|---|
| Humidity experiment | 3% or less | USP, EP |

[Table 8]

| Vial No. | Time (h) | Humidity (%) |
|---|---|---|
| # 25 | 0.5 | 1.01 |
| # 26 | 1 | 1.53 |
| #27 | 4 | 1.49 |

As shown in Table 8, the humidity of the botulinum toxin formulation after the drying process for 0.5 to 4 hours satisfied the range shown in Table 7 above.

**Example 3. Drying time under the reduced pressure drying conditions of the present invention #2**

Based on the results of Example 2, the empty vials were filled with the final stock solution of the botulinum toxin prepared in the above preparation example by 0.1 mL each, and time for the phase change at each pressure condition was examined.

Specifically, as shown in Table 9 below, the drying process was performed for 3 hours at conditions of 50,000 to 70,000 mTorr and 25°C. Change in temperature due to the phase change was measured by a temperature sensor installed in the vial. The speed at which the phase change occurs was faster with lower pressure, by which it was confirmed that evaporation was occurring.

[Table 9]

| Vial No. | Pressure (mTorr) | Time for phase change (min) |
|---|---|---|
| # 28 | 50,000 | 143 |
| # 29 | 60,000 | 183 |
| # 30 (Comparative Example 8) | 70,000 | - |

As shown in Table 9 and Fig. 4, the time taken for the phase change to occur under 50,000 and 60,000 mTorr conditions was 143 minutes and 183 minutes, respectively. However, at 70,000 mTorr, this phenomenon was not observed.

The above results suggest that at higher pressures, the time required for complete drying (evaporation) will increase, and that preparation of the botulinum toxin dried cake pursued by the present invention may be difficult at pressures exceeding a certain range (Comparative Example 8).

## Claims

1. A method for preparing a botulinum toxin dried cake, comprising the step of drying the botulinum toxin under reduced pressure at pressure conditions of 1,500 mTorr to 60,000 mTorr.

2. The method of claim 1,
**characterized in that** the drying under reduced pressure is performed at temperature conditions of 3°C to 25°C.

3. The method of claim 1 or 2,
**characterized in that** the drying under reduced pressure is performed for 0.5 hours or more, or 0.5 to 4 hours.

4. The method according to any one of claims 1 to 3,
**characterized in that** the concentration of the botulinum toxin is 250 U/mL to 5,000 U/mL.

5. The method according to any one of claims 1 to 4,
**characterized in that** the produced botulinum toxin dried cake has a standard titer of 80% to 120%, or 85% to 115%.

6. The method according to any one of claims 1 to 5,
**characterized in that** drying under reduced pressure is performed until the humidity of the botulinum toxin dried cake reaches 3% or less.

7. The method according to any one of claims 1 to 6,
**characterized in that** the strain producing botulinum toxin is *Clostridium botulinum (C. botulinum)* type A.

8. The method according to any one of claims 1 to 7,
**characterized in that** the pressure conditions for drying under reduced pressure are determined by reaching the target pressure at a constant speed in a stably controlled state from normal pressure.

9. The method according to any one of claims 1 to 8,
**characterized in that** the drying under reduced pressure is performed at a pressure of 1,500 to 60,000 mTorr, 1,500 to 55,000 mTorr, 1,500 to 50,000 mTorr, 1,500 to 45,000 mTorr, 1,500 to 40,000 mTorr, 1,500 to 35,000 mTorr, 1,500 to 30,000 mTorr, 1,500 to 25,000 mTorr, 1,500 to 20,000 mTorr, 1,500 to 15,000 mTorr, 1,500 to 14,000 mTorr, 1,500 to 13,000 mTorr, 1,500 to 12,000 mTorr, 1,500 to 11,000 mTorr, 1,500 to 10,000 mTorr, 1,500 to 9,000 mTorr, 1,500 to 8,000 mTorr, 1,500 to 7,000 mTorr, 1,500 to 6,000 mTorr, 1,500 to 5,000 mTorr, 1,500 to 4,000 mTorr, 1,500 to 3,500 mTorr, 1,500 to 3,000 mTorr, 1,500 to 2,500 mTorr, 1,500 to 2,000 mTorr, 2,500 to 3,000 mTorr, 2,500 to 3,500 mTorr, 2,500 to 4,000 mTorr, 2,500 to 4,500 mTorr, 2,500 to 5,000 mTorr, 2,500 to 6,000 mTorr, 2,500 to 7,000 mTorr, 2,500 to 8,000 mTorr, 2,500 to 9,000 mTorr, 2,500 to 10,000 mTorr, 2,500 to 11,000 mTorr, 2,500 to 12,000 mTorr, 2,500 to 13,000 mTorr, 2,500 to 14,000 mTorr, 2,500 to 15,000 mTorr, 2,500 to 20,000 mTorr, 2,500 to 25,000 mTorr, 2,500 to 30,000 mTorr, 2,500 to 35,000 mTorr, 2,500 to 40,000 mTorr, 2,500 to 45,000 mTorr, 2,500 to 50,000 mTorr, 2,500 to 55,000 mTorr, or 2,500 to 60,000 mTorr.

10. The method according to any one of claims 1 to 9,
**characterized in that** the drying under reduced pressure is performed at a temperature of 5°C to 25°C, 7°C to 25°C, 9°C to 25°C, 11°C to 25°C, 12°C to 25°C, 3°C to 20°C, 5°C to 20°C, 7°C to 20°C, 9°C to 20°C, 11°C to 20°C, 12°C to 20°C, 3°C to 18°C, 3°C to 16°C, 3°C to 14°C, or 3°C to 12°C.

11. The method according to any one of claims 1 to 10,
**characterized in that** the drying under reduced pressure is performed in the range of 0.5 hours to 4 hours, 0.5 hours to 3 hours, 0.5 hours to 2 hours, 0.5 hours to 1 hours, 1 hours to 4 hours, 2 hours to 4 hours, or 3 hours to 4 hours.

Fig. 1

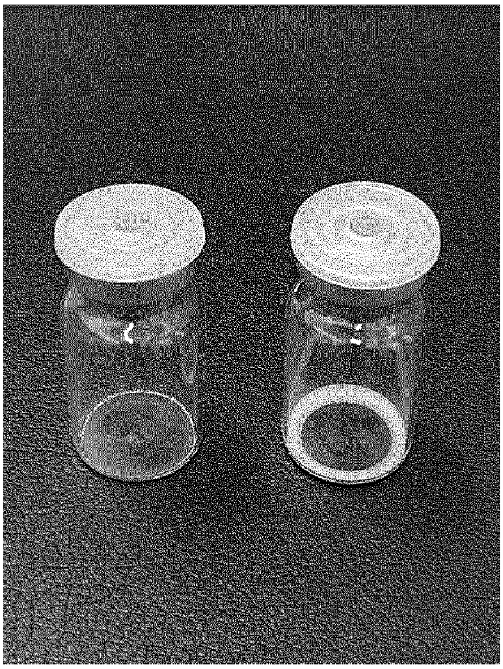

Fig. 2a

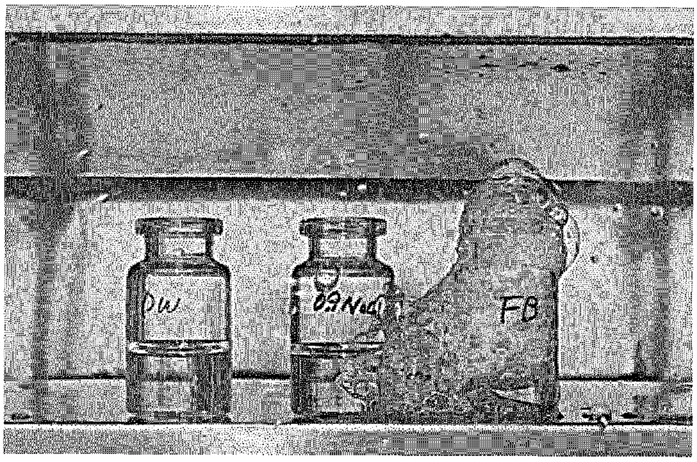


Fig. 2b

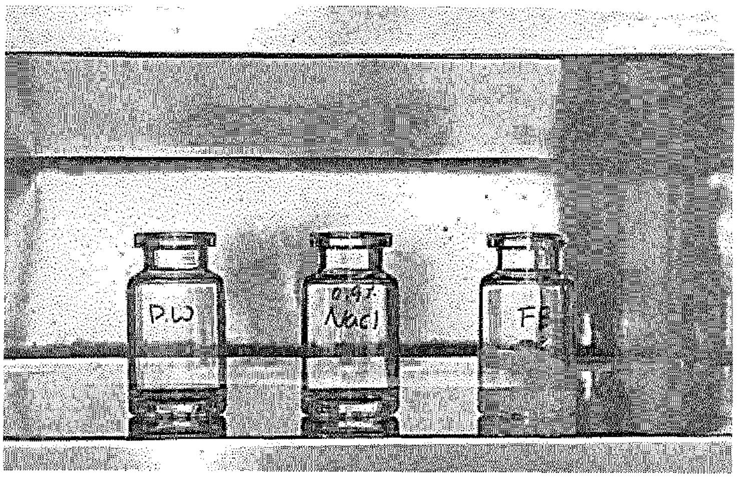

Fig. 3a

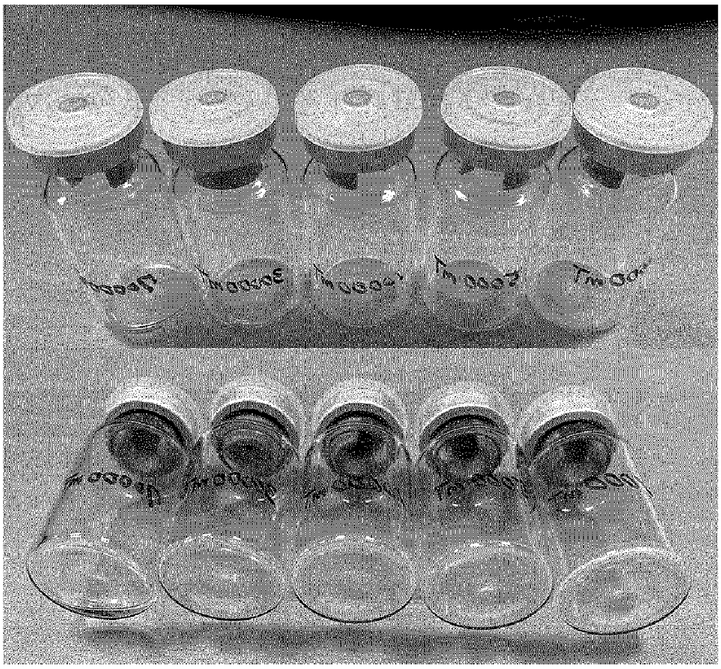

Fig. 3b

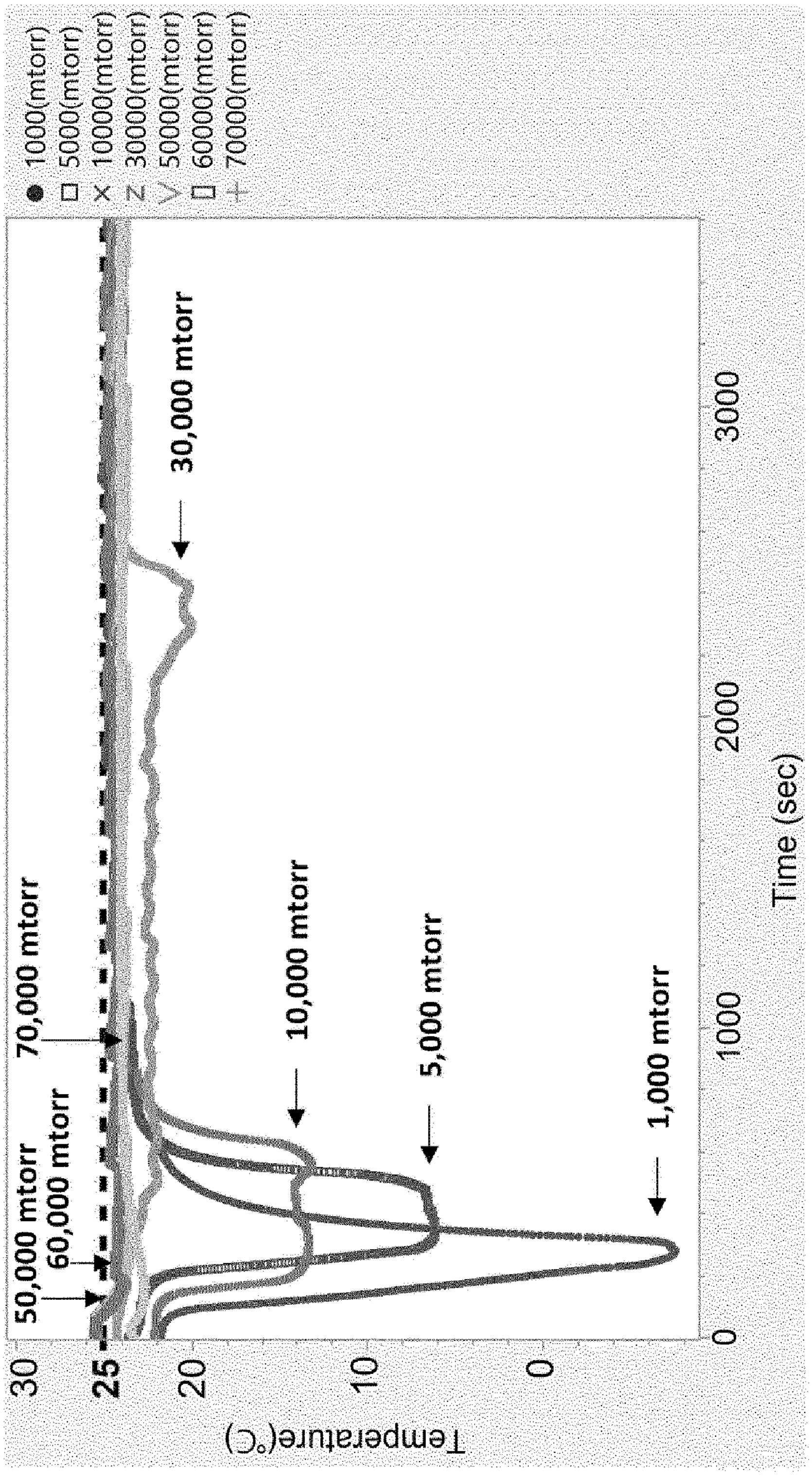
1000(mtorr)
5000(mtorr)
10000(mtorr)
30000(mtorr)
50000(mtorr)
60000(mtorr)
70000(mtorr)
Temperature(°C)
30
25
20
10
0
Time (sec)
0
1000
2000
3000
50,000 mtorr
60,000 mtorr
70,000 mtorr
10,000 mtorr
5,000 mtorr
1,000 mtorr
30,000 mtorr

Fig. 4

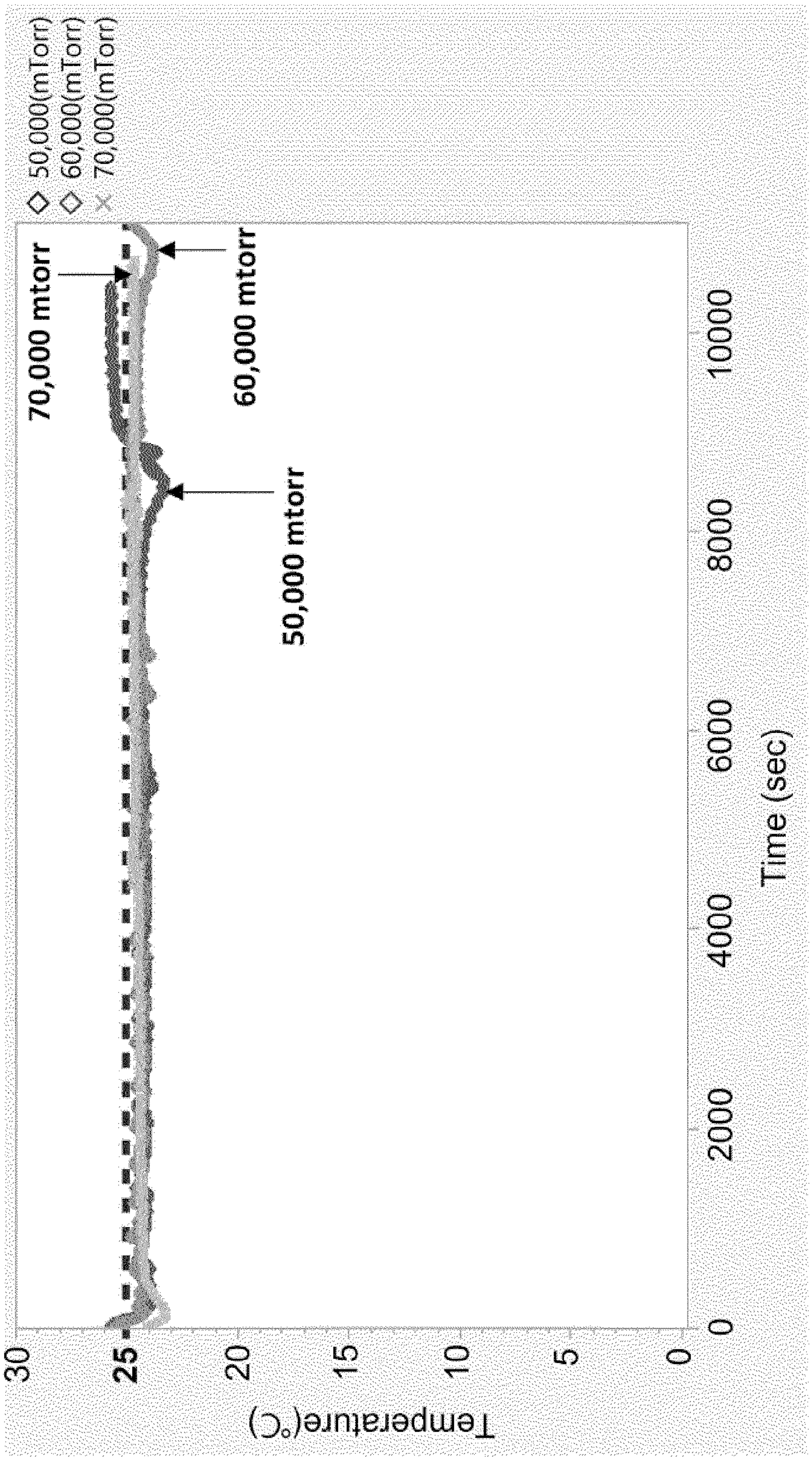
50,000(mTorr)
60,000(mTorr)
70,000(mTorr)
70,000 mtorr
60,000 mtorr
50,000 mtorr
Temperature(°C)
30
25
20
15
10
5
0
Time (sec)
0
2000
4000
6000
8000
10000

## INTERNATIONAL SEARCH REPORT

International application No.
**PCT/KR2022/010991**

**A. CLASSIFICATION OF SUBJECT MATTER**

**C12N 9/64**(2006.01)i; **C07K 14/33**(2006.01)i; **A61K 9/16**(2006.01)i; **A61K 38/48**(2006.01)i; **A61K 8/02**(2006.01)i; **A61K 8/66**(2006.01)i

According to International Patent Classification (IPC) or to both national classification and IPC

**B. FIELDS SEARCHED**

Minimum documentation searched (classification system followed by classification symbols)

C12N 9/64(2006.01); A61K 38/16(2006.01); A61K 39/145(2006.01); A61K 47/26(2006.01); A61K 47/34(2006.01); A61K 8/99(2006.01); A61K 9/14(2006.01); C07K 1/18(2006.01); C07K 14/33(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 보툴리눔(botulinum), 독소(toxin), 감압건조(reduced pressure drying), 건조(dry)

**C. DOCUMENTS CONSIDERED TO BE RELEVANT**

| Category* | Citation of document, with indication, where appropriate, of the relevant passages | Relevant to claim No. |
|---|---|---|
| A | KR 10-2017-0019489 A (ALLERGAN, INC.) 21 February 2017 (2017-02-21) See paragraph [0087]. | 1-11 |
| A | KR 10-2017-0133530 A (REVANCE THERAPEUTICS, INC.) 05 December 2017 (2017-12-05) See paragraphs [0026] and [0074]-[0087]. | 1-11 |
| A | KR 10-2011-0007099 A (SANOFI PASTEUR) 21 January 2011 (2011-01-21) See paragraphs [0044] and [0073]-[0074]. | 1-11 |
| A | KR 10-2012-0022777 A (MERZ PHARMA GMBH & CO. KGAA) 12 March 2012 (2012-03-12) See paragraphs [0076]-[0086]. | 1-11 |
| A | KR 10-2007-0116710 A (ALLERGAN, INC.) 11 December 2007 (2007-12-11) See paragraph [0080]. | 1-11 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **31 October 2022** | **01 November 2022** |

| Name and mailing address of the ISA/KR | Authorized officer |
|---|---|
| **Korean Intellectual Property Office Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
**Information on patent family members**

International application No.
**PCT/KR2022/010991**

| Patent document cited in search report | Publication date (day/month/year) | Patent family member(s) | Publication date (day/month/year) |
|---|---|---|---|
| KR 10-2017-0019489 A | 21 February 2017 | AU 2010-273601 A1 | 02 February 2012 |
| | | AU 2010-273601 A1 | 20 January 2011 |
| | | BR 112012000784 A2 | 11 August 2020 |
| | | CA 2767760 A1 | 20 January 2011 |
| | | CA 2767760 C | 04 December 2018 |
| | | CN 102482332 A | 30 May 2012 |
| | | CN 102482332 B | 03 August 2016 |
| | | CN 106117325 A | 16 November 2016 |
| | | CY 1119519 T1 | 07 March 2018 |
| | | DK 2454275 T3 | 23 October 2017 |
| | | DK 3252070 T3 | 20 January 2020 |
| | | EP 2454275 A1 | 23 May 2012 |
| | | EP 3252070 A1 | 06 December 2017 |
| | | EP 3640259 A1 | 22 April 2020 |
| | | ES 2643554 T3 | 23 November 2017 |
| | | ES 2770031 T3 | 30 June 2020 |
| | | HK 1247629 A1 | 28 September 2018 |
| | | HU E033888 T2 | 29 January 2018 |
| | | HU E047362 T2 | 28 April 2020 |
| | | IN 456DEN2012 A | 15 May 2015 |
| | | JP 2012-532932 A | 20 December 2012 |
| | | JP 2016-028601 A | 03 March 2016 |
| | | JP 2017-070291 A | 13 April 2017 |
| | | JP 2020-078306 A | 28 May 2020 |
| | | JP 2021-193103 A | 23 December 2021 |
| | | KR 10-2015-0085140 A | 22 July 2015 |
| | | KR 10-2018-0122039 A | 09 November 2018 |
| | | KR 10-2019-0111161 A | 01 October 2019 |
| | | KR 10-2012105417 A | 25 September 2012 |
| | | MX 2012000615 A | 26 March 2012 |
| | | PL 2454275 T3 | 29 December 2017 |
| | | PL 3252070 T3 | 30 April 2020 |
| | | PT 2454275 T | 23 October 2017 |
| | | PT 3252070 T | 14 January 2020 |
| | | RU 2012104778 A | 20 August 2013 |
| | | RU 2561459 C2 | 27 August 2015 |
| | | SI 2454275 T1 | 29 December 2017 |
| | | SI 3252070 T1 | 31 March 2020 |
| | | US 2011-0008843 A1 | 13 January 2011 |
| | | US 2012-0108792 A1 | 03 May 2012 |
| | | US 2012-0123095 A1 | 17 May 2012 |
| | | US 2012-0156756 A1 | 21 June 2012 |
| | | US 2012-0245324 A1 | 27 September 2012 |
| | | US 2015-0184141 A1 | 02 July 2015 |
| | | US 2016-0097045 A1 | 07 April 2016 |
| | | US 2017-327811 A1 | 16 November 2017 |
| | | US 2020-071686 A1 | 05 March 2020 |
| | | US 2021-292727 A1 | 23 September 2021 |
| | | US 2022-033796 A1 | 03 February 2022 |
| | | WO 2011-008713 A1 | 20 January 2011 |

Form PCT/ISA/210 (patent family annex) (July 2022)

**INTERNATIONAL SEARCH REPORT**
**Information on patent family members**

International application No.
**PCT/KR2022/010991**

| Patent document cited in search report | Publication date (day/month/year) | Patent family member(s) | Publication date (day/month/year) |
|---|---|---|---|
| KR 10-2017-0133530 A | 05 December 2017 | AU 2010-265888 A1 | 19 January 2012 |
| | | AU 2010-265888 A1 | 29 December 2010 |
| | | BR PI1015938 A2 | 27 September 2016 |
| | | CA 2766521 A1 | 29 December 2010 |
| | | CA 2766521 C | 21 July 2020 |
| | | CN 102869373 A | 09 January 2013 |
| | | CO 6491076 A2 | 31 July 2012 |
| | | EP 2445521 A2 | 02 May 2012 |
| | | IL 217156 A | 29 February 2012 |
| | | IL 217156 D0 | 29 February 2012 |
| | | IL 268980 A | 31 October 2019 |
| | | IL 268980 D0 | 31 October 2019 |
| | | JP 2012-531442 A | 10 December 2012 |
| | | KR 10-2012-0102569 A | 18 September 2012 |
| | | KR 10-2019-0047736 A | 08 May 2019 |
| | | KR 10-2020-00141546 A | 18 December 2020 |
| | | KR 10-2021-0141783 A | 23 November 2021 |
| | | MX 2012000174 A | 23 May 2012 |
| | | MX 2019008126 A | 09 September 2019 |
| | | SG 177357 A1 | 28 February 2012 |
| | | US 2010-0330123 A1 | 30 December 2010 |
| | | US 2012-0107361 A1 | 03 May 2012 |
| | | US 2016-0256532 A1 | 08 September 2016 |
| | | US 2019-290740 A1 | 26 September 2019 |
| | | US 2021-330766 A1 | 28 October 2021 |
| | | WO 2010-151840 A2 | 29 December 2010 |
| | | WO 2010-151840 A3 | 20 March 2014 |
| KR 10-2011-0007099 A | 21 January 2011 | AR 070800 A1 | 05 May 2010 |
| | | AU 2009-221180 A1 | 11 September 2009 |
| | | AU 2013-203067 A1 | 02 May 2013 |
| | | BR PI0909820 A2 | 11 August 2015 |
| | | CA 2716399 A1 | 11 September 2009 |
| | | CA 2716399 C | 21 July 2020 |
| | | CN 101959527 A | 26 January 2011 |
| | | CN 105944095 A | 21 September 2016 |
| | | EP 2254594 A1 | 01 December 2010 |
| | | EP 2589392 A2 | 08 May 2013 |
| | | EP 2589392 A3 | 21 August 2013 |
| | | ES 2615390 T3 | 06 June 2017 |
| | | IL 207308 A | 30 December 2010 |
| | | IL 207308 D0 | 30 December 2010 |
| | | JP 2011-514899 A | 12 May 2011 |
| | | MX 2010008799 A | 07 September 2010 |
| | | US 2009-0232894 A1 | 17 September 2009 |
| | | US 2014-0154329 A1 | 05 June 2014 |
| | | US 2016-0235831 A1 | 18 August 2016 |
| | | WO 2009-109550 A1 | 11 September 2009 |
| | | ZA 201006225 B | 30 November 2011 |
| KR 10-2012-0022777 A | 12 March 2012 | AU 2010-237270 A1 | 21 October 2010 |
| | | AU 2010-237270 A1 | 20 October 2011 |

Form PCT/ISA/210 (patent family annex) (July 2022)

**INTERNATIONAL SEARCH REPORT**
**Information on patent family members**

International application No.
**PCT/KR2022/010991**

| Patent document cited in search report | Publication date (day/month/year) | Patent family member(s) | Publication date (day/month/year) |
|---|---|---|---|
| | | BR PI1014034 A2 | 12 April 2016 |
| | | CA 2758754 A1 | 21 October 2010 |
| | | CN 102387791 A | 21 March 2012 |
| | | CN 107789620 A | 13 March 2018 |
| | | EP 2248518 A1 | 10 November 2010 |
| | | EP 2419089 A1 | 22 February 2012 |
| | | ES 2606567 T3 | 24 March 2017 |
| | | HK 1248556 A1 | 19 October 2018 |
| | | IL 215354 A | 29 December 2011 |
| | | IL 215354 D0 | 29 December 2011 |
| | | IN 4251KON2011 A | 10 July 2015 |
| | | JP 2012-524035 A | 11 October 2012 |
| | | MX 2011010801 A | 28 October 2011 |
| | | RU 2011146541 A | 27 May 2013 |
| | | RU 2539388 C2 | 20 January 2015 |
| | | US 2012-0093866 A1 | 19 April 2012 |
| | | US 2016-0184413 A1 | 30 June 2016 |
| | | WO 2010-118888 A1 | 21 October 2010 |
| | | ZA 201108433 B | 26 June 2013 |
| KR 10-2007-0116710 A | 11 December 2007 | AR 045813 A1 | 16 November 2005 |
| | | AT 469215 T | 15 June 2010 |
| | | AU 2004-280450 A1 | 21 April 2005 |
| | | AU 2004-280450 B2 | 05 February 2009 |
| | | AU 2005-293288 A1 | 20 April 2006 |
| | | AU 2005-327457 A1 | 05 October 2006 |
| | | AU 2005-327457 B2 | 23 August 2012 |
| | | AU 2005-327458 A1 | 05 October 2006 |
| | | AU 2005-327458 B2 | 15 December 2011 |
| | | AU 2006-295305 A1 | 05 April 2007 |
| | | AU 2006-295305 B2 | 31 January 2013 |
| | | AU 2012-201519 A1 | 05 April 2012 |
| | | AU 2012-201519 B2 | 26 June 2014 |
| | | AU 2013-202885 A1 | 02 May 2013 |
| | | AU 2013-204742 A1 | 09 May 2013 |
| | | BR PI0414722 A | 21 November 2006 |
| | | BR PI0508299 A | 31 July 2007 |
| | | BR PI0508374 A | 31 July 2007 |
| | | BR PI0616041 A2 | 07 June 2011 |
| | | CA 2540072 A1 | 21 April 2005 |
| | | CA 2556537 A1 | 03 September 2006 |
| | | CA 2556796 A1 | 03 September 2006 |
| | | CA 2556796 C | 23 January 2018 |
| | | CA 2602440 A1 | 05 April 2007 |
| | | CA 2850858 A1 | 05 April 2007 |
| | | CN 101119688 A | 06 February 2008 |
| | | CN 1856569 A | 01 November 2006 |
| | | CN 1856569 C | 01 November 2006 |
| | | CN 1930186 A | 14 March 2007 |
| | | CN 1930186 C | 14 March 2007 |
| | | CN 1946738 A | 11 April 2007 |

Form PCT/ISA/210 (patent family annex) (July 2022)

**INTERNATIONAL SEARCH REPORT**
**Information on patent family members**

International application No.
**PCT/KR2022/010991**

| Patent document cited in search report | Publication date (day/month/year) | Patent family member(s) | Publication date (day/month/year) |
|---|---|---|---|
| | | DK 1664292 T3 | 30 August 2010 |
| | | DK 1853623 T3 | 15 July 2013 |
| | | EP 1396243 A1 | 10 March 2004 |
| | | EP 1664292 A2 | 07 June 2006 |
| | | EP 1827325 A1 | 05 September 2007 |
| | | EP 1853622 A1 | 14 November 2007 |
| | | EP 1853623 A1 | 14 November 2007 |
| | | EP 1924292 A2 | 28 May 2008 |
| | | EP 2177607 A1 | 21 April 2010 |
| | | EP 2335658 A1 | 22 June 2011 |
| | | ES 2344843 T3 | 08 September 2010 |
| | | ES 2420430 T3 | 23 August 2013 |
| | | ES 2509872 T3 | 20 October 2014 |
| | | IL 177467 A | 30 September 2013 |
| | | IL 177467 D0 | 20 March 2008 |
| | | IL 177468 A | 20 March 2008 |
| | | IL 177468 D0 | 20 March 2008 |
| | | IL 227909 A | 30 September 2013 |
| | | IL 227909 D0 | 30 September 2013 |
| | | IL 241873 A | 30 November 2015 |
| | | IL 241873 D0 | 30 November 2015 |
| | | JP 2007-506427 A | 22 March 2007 |
| | | JP 2008-515582 A | 15 May 2008 |
| | | JP 2008-531046 A | 14 August 2008 |
| | | JP 2008-531047 A | 14 August 2008 |
| | | JP 2009-508857 A | 05 March 2009 |
| | | JP 2012-041356 A | 01 March 2012 |
| | | JP 2012-070759 A | 12 April 2012 |
| | | JP 2012-125251 A | 05 July 2012 |
| | | JP 2013-014600 A | 24 January 2013 |
| | | JP 2015-051019 A | 19 March 2015 |
| | | KR 10-2006-0102330 A | 27 September 2006 |
| | | KR 10-2007-0104334 A | 25 October 2007 |
| | | KR 10-2007-0115580 A | 06 December 2007 |
| | | MX PA06003029 A | 23 June 2006 |
| | | MX PA06009853 A | 29 March 2007 |
| | | MX PA06009854 A | 29 March 2007 |
| | | NZ 545503 A | 30 April 2009 |
| | | PL 1664292 T3 | 29 October 2010 |
| | | TW 200516142 A | 16 May 2005 |
| | | US 2005-0069562 A1 | 31 March 2005 |
| | | US 2005-0143765 A1 | 30 June 2005 |
| | | US 2005-0143766 A1 | 30 June 2005 |
| | | US 2005-0238668 A1 | 27 October 2005 |
| | | US 2005-0238669 A1 | 27 October 2005 |
| | | US 2005-0251182 A1 | 10 November 2005 |
| | | US 2006-0228777 A1 | 12 October 2006 |
| | | US 2006-0228780 A1 | 12 October 2006 |
| | | US 2006-0240514 A1 | 26 October 2006 |
| | | US 2007-0066541 A1 | 22 March 2007 |

Form PCT/ISA/210 (patent family annex) (July 2022)

**INTERNATIONAL SEARCH REPORT**
**Information on patent family members**

International application No.
**PCT/KR2022/010991**

| Patent document cited in search report | Publication date (day/month/year) | Patent family member(s) | Publication date (day/month/year) |
|---|---|---|---|
| | | US 2007-0111288 A1 | 17 May 2007 |
| | | US 2008-0027469 A1 | 31 January 2008 |
| | | US 2009-0022763 A1 | 22 January 2009 |
| | | US 2009-0123497 A1 | 14 May 2009 |
| | | US 2009-0124790 A1 | 14 May 2009 |
| | | US 2009-0274729 A1 | 05 November 2009 |
| | | US 2010-0222587 A1 | 02 September 2010 |
| | | US 2011-0152608 A1 | 23 June 2011 |
| | | US 2012-0149089 A1 | 14 June 2012 |
| | | US 2013-0171716 A1 | 04 July 2013 |
| | | US 2014-0039250 A1 | 06 February 2014 |
| | | US 2014-0178966 A1 | 26 June 2014 |
| | | US 2017-342395 A1 | 30 November 2017 |
| | | US 2021-024913 A1 | 28 January 2021 |
| | | US 2021-079370 A1 | 18 March 2021 |
| | | US 2021-222142 A1 | 22 July 2021 |
| | | US 2021-238572 A1 | 05 August 2021 |
| | | WO 2005-035749 A2 | 21 April 2005 |
| | | WO 2005-035749 A3 | 02 June 2005 |
| | | WO 2006-040647 A1 | 20 April 2006 |
| | | WO 2006-096163 A1 | 14 September 2006 |
| | | WO 2006-096164 A1 | 14 September 2006 |
| | | WO 2007-037849 A2 | 05 April 2007 |
| | | WO 2007-037849 A3 | 24 January 2008 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020120112248 A